# EUROPEAN PATENT APPLICATION

(11) **EP 2 161 048 A1**
(43) Date of publication of application: **10.03.2010**
(21) Application number: 09169567.6
(22) Date of filing: 07.09.2009
(51) Int. Cl.: A61M 25/04, A61B 17/00, A61F 2/24

(54) **Composite device for controlling the mitral valve plastics operation and providing a simultaneous cardioplegic infusion in a conservative surgical operation on the mitral valve**

(30) Priority: 09.09.2008 IT MI20081601
(71) Applicant: N.G.C. Medical S.p.A., 22060 Novedrate CO (IT)
(72) Inventor: Patane', Francesco, I-22060, Novedrate (COMO) (IT); Pini, Patrizia, I-22060, Novedrate (COMO) (IT); Cremascoli, Eugenio, I-22060, Novedrate (COMO) (IT)
(74) Representative: Cicogna, Franco

(57) **Abstract**

A composite device for controlling a mitral valve plastics repairing operation and providing a simultaneous cardioplegia solution infusion in a conservative surgical repairing operation on a mitral valve comprises two coupled catheters, one for performing an infusion of a physiologic solution for filling-in a heart ventricle, and the other for providing an aorta anchoring and operating as an access tip part for cardioplegia, ventricular filling and the like infusion solutions.

## Description

### BACKGROUND OF THE INVENTION

The present invention relates to a composite device for controlling the mitral valve plastics operation and providing a simultaneous cardioplegic infusion in a conservative surgical operation on the mitral valve.

As is known the mitral valve is a cardiac valve arranged between the left auricle and left ventricle.

The oxygenated blood flowing from lungs during the diastolic phase is collected in the left auricle and hence in the left ventricle through the mitral valve.

During the systolic phase, the blood, from the left auricle and through the aortic valve, will perfuse through the overall body.

The mitral valve prevents the ventricle blood from returning to the heart auricle.

The mitral valve comprises two valve flaps or edge portions, coupled to the auricle papillary muscles through the tendinous cords, and perfectly operates as the two valve flaps or edge portions are fully closed.

On the contrary, it is stenotic if it is not able of properly opening and it is considered of insufficient performance if it does not fully close.

As the mitral valve is damaged, it is necessary to perform a surgical repairing operation thereon.

Such an operation can be either a replacement one, in which the natural mitral valve is replaced by an artificial mitral valve, or of a conservative type, as the natural valve is repaired.

In recent years, the surgical treatment of mitral valve damages is nearly exclusively of a conservative type or nature.

This is advantageous for several reasons: in fact, the operated patient is not subjected to a chronic anticoagulant therapy; the natural mitral valve has a much greater resistance to infections; moreover, the left auricle function is more safely preserved.

The surgical procedures used for repairing insufficient mitral valves are of different types, depending on the type of plastics operations to be performed.

The most part of mitral valve repairing plastics operations performed according to a non invasive surgical method, that is a bypass heart-lung operation performed by an extracorporeal circulation procedure (CEC).

During such an extracorporeal circulation, the patient heart and lungs are stopped and the patient natural perfusion is hindered by aortic clamping means, whereas mechanical perfusion is carried through a cannula system, a pump and an oxygenating device including a heat exchanger.

The heart stop should not cause ischemic insults or damages to the cardiac muscle.

This result is obtained by evenly infusing or introducing into the coronary arteries of the patient a solution comprising oxygenated blood, potassium chloride and magnesium sulphate.

This solution, the so-called "cardioplegia solution" allows a stopped or non beating heart to be properly oxygenated.

Alternatively, it is possible to administer to the patient only patient oxygenated blood (the so-called haematic cardioplegia).

Different procedures for administering cardioplegia solutions are known, in particular one of an anterograde type if the cardioplegia solution is administered by following the coronary artery physiologic path, or of a retrograde type, if it is administered by following the reverse administering path.

The anterograde procedure or method is broadly diffused, and it is carried out by using specifically constructed cannulas, the most common of which comprises a two-way system, the two ways of which converge to an invasive pointed or end tip assembly, a feeding line or way being used for administering the cardioplegia solution, and the other line or way being used for performing a deaerating process on the left heart cavities; the flange of the pointed or end tip assembly being affixed at fixation points to the aorta, at the level of the aortic root and upstream of the aortic clamped region, whereas the pointed or tip portion penetrates the aorta to carry out its target operations.

Based on preoperating data, a surgeon evaluates the possibility of performing a mitral valve plastics operation, and such a possibility and its possible result are evaluated before CEC by a transesophageal electrocardiogram (ECG).

That same mode of operation or mechanism is used at the end of the CEC for validating the plastics operation result.

In a case of a not satisfactory result, the patient is subjected again to a CEC, and the operation is carried out again.

To reduce to a minimum the need of performing a new CEC, cardiosurgeons conventionally fill-in the left ventricular cavity with a physiologic solution, to evaluate, by view, the mitral valve tightness capability.

This operation is performed by injecting a physiologic solution from a physiologic solution injecting syringe into a draining tube which, from the left auricle, passes through the mitral valve to be introduced into the left ventricle.

The above physiologic solution fills-in the ventricle and puts the mitral valve under tension: a possible leakage of the valve causing a leakage of the physiology solution to the auricle, which leakage can be easily seen by the surgeon.

However, the above disclosed method is not a reliable one, since the provision of said draining tube prevents the valve flaps or edge portions from perfectly engaging with one another.

Another procedure used for monitoring a proper result of the mitral valve plastics operation is that of filling-in the left ventricular cavity through the aortic valve by using a draining assembly which is engaged from the aorta.

The need of providing a short aortotomy, however, has the disadvantages of extending the aorta clamping and CEC times, thereby increasing the risks of bleeding and related complications.

### SUMMARY OF THE INVENTION

Accordingly, the aim of the present invention is to provide such a device allowing to anatomically test the mitral valve at the end of a mitral valve plastics repairing operation, as well as during a CEC operation, through a filling-in of the left ventricle.

Within the scope of the above mentioned aim, a main object of the invention is to provide such a device which does not pass through a mitral valve and, moreover, does not require a further aortotomy operation.

Yet another object of the present invention is to provide such a device which, in performing a mitral valve repairing plastics operation, does not require to form several catheter introducing places.

Yet another object of the invention is to provide such a device assuring a very high reliability of the repaired mitral valve tightness test.

Yet another object of the present invention is to provide such a device allowing to perform very quick and reliable monitoring operations at the end of the mitral valve repairing plastics operation.

Yet another object of the invention is to provide such a device adapted to greatly simplify the operation of the monitoring system while allowing to use other device conventionally provided for performing mitral valve repairing operations.

According to one aspect of the present invention, the above mentioned aim and objects, as well as yet other objects, which will become more apparent hereinafter, are achieved by a composite device for controlling a mitral valve repairing plastics operation and providing a simultaneous cardioplegia solution infusion in a conservative surgical operation on the mitral valve, **characterized in that** said composite device comprises two catheters coupled to one another, one of said catheters being used for infusing a physiologic solution for filling-in a heat ventricle, the other catheter being used for providing an aorta anchoring and having an access tip suitable for different types of infusions such as a cardioplegia solution infusion and a ventricular filling-in infusion.

### BRIEF DESCRIPTION OF THE DRAWINGS

Further characteristics and advantages of the present invention will become more apparent hereinafter from the following detailed disclosure of a preferred, though not exclusive, embodiment of the invention, which is illustrated, by way of an indicative, but not limitative, example in the accompanying drawings, where:

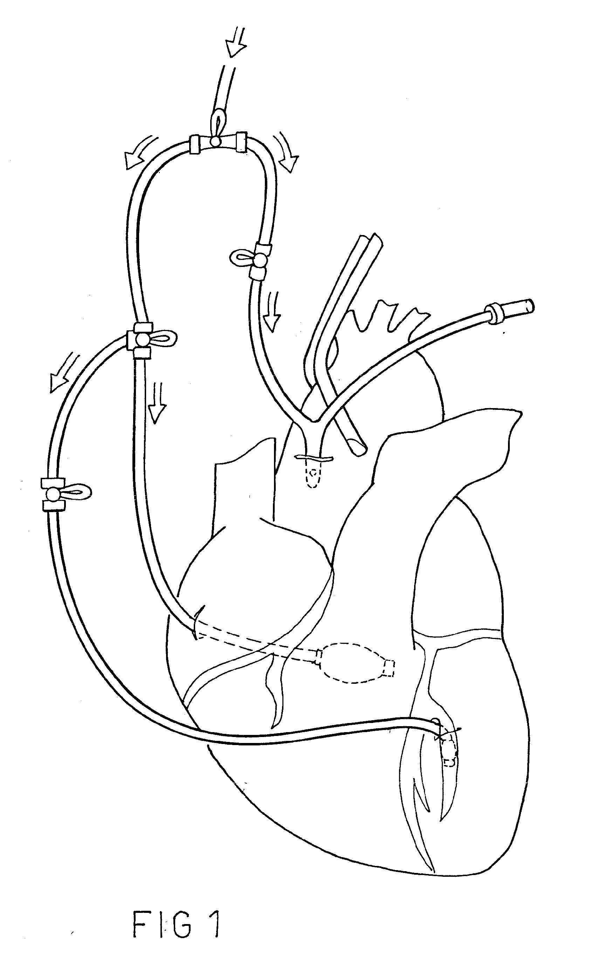

Figure 1 schematically shows the system for administering a cardioplegia infusion, of an anterograde and retrograde type, being used in a mitral valve plastics conservative surgical operation;

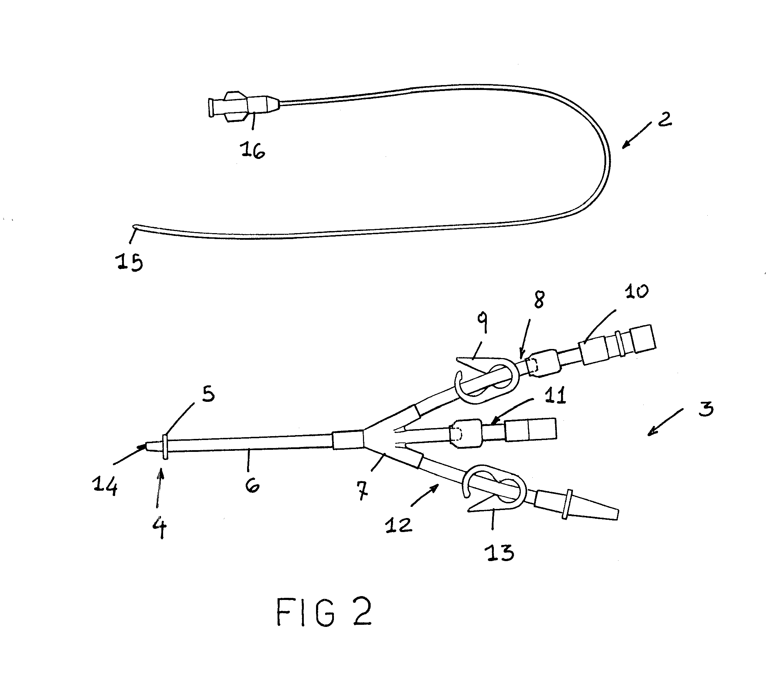

Figure 2 is a perspective view of the composite device for controlling or monitoring the mitral valve plastics operation and a simultaneous infusion of a cardioplegia solution according to the present invention;
Figure 3 show a starting step for locating the inventive system, providing to install a cannula into the aorta, upstream of the aorta clamped region, or in the aorta root;
Figure 4 shows the cannula in its proper position, with the introduced catheter, and the connected cardioplegia system;
Figure 5 shows the cannula in its position, with the catheter at a retracted or withdrawn position, and with a connected cardioplegia system;
Figure 6 shows the device or system with the catheter well engaged in the ventricle and in an operating condition, while injecting a cardioplegia solution through an injection syringe; and
Figure 7 shows the system including a catheter of a "pig tail" type.

### DESCRIPTION OF THE PREFERRED EMBODIMENTS

With reference to the number references of the above mentioned figures, the device according to the present invention, which has been generally indicated by the reference number 1, comprises two catheters coupled to one another: one of said catheters, indicated by the reference number 2, being used for infusing a physiologic solution for filling-in the heart ventricle, the other catheter, indicated by the reference number 3, being designed for performing an aorta anchoring step and having an access tip portion for accessing different types of infusion systems (cardioplegia and ventricular filling-in infusions).

The second catheter 3 is constituted by a cannula used for accessing the aorta and comprises:
- an implantable (tip) part 4, made of a soft material (such as PVC, PU or the like), with a flange 5 for suturing said part to the aorta vessel, and having a suitable target diameter (for example 3 mm);
- a cannula body 6 made of a flexible material (such as PVC, PU or the like), of a single-volume type, thereby allowing to perform a direct connection to the tip part 4;
- a fitting or coupling region 7 for the three vessel access ways;
- a ventricular filling-in line 8, with a clamping element 9 and a haemostatic valve 10;
- a cardioplegia line 11, to be connected to the cardioplegia solution circuit;
- a venting line 12, with a related clamping element 13;
- an aorta piercing needle 14, of an occlusive type with respect to the tip 4.

The three ways or lumens of the cannula can be mutually reversed, that is they are not defined in an absolute fixed manner, but are provided for operating according to the connections provided for the outer system lines: for example the cardioplegia line 11 may be arranged on a side and the filling-in line 8 may have a straight or linear configuration.

The mitral valve testing catheter 2 is a single volume extruded element made of a soft and strong material (polyetherblockamide, PU or the like) and has a thin thickness; the point or tip portion 15 thereof being atraumatical and having a diameter less than that of the tip 4 of the cannula 3 (for example of 2 mm) and being suitable perforated.

By a standard luer connection element 16, said catheter can be directly coupled to an infusion syringe 17 for properly infusing the physiologic solution.

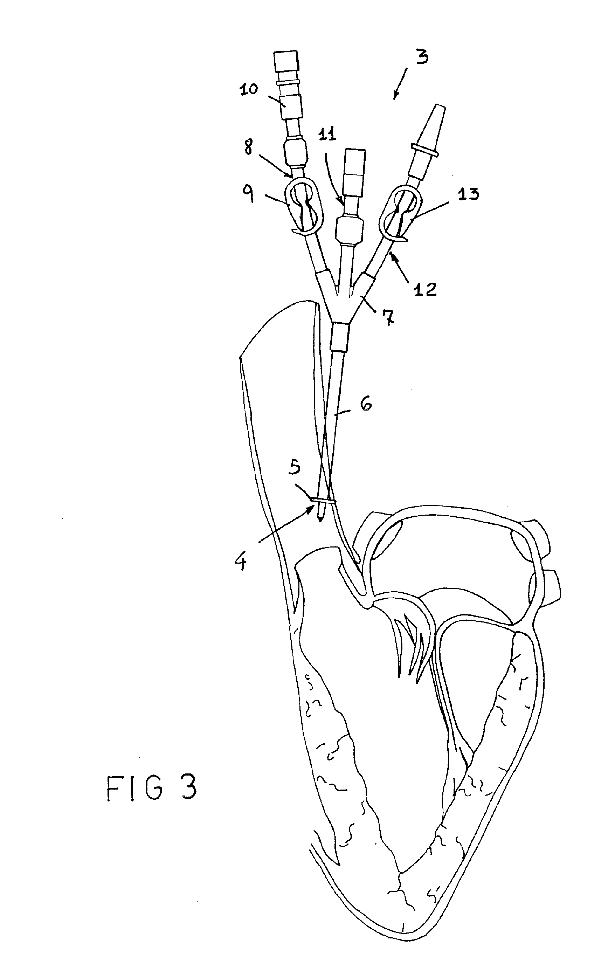

The operation for properly locating the subject system, as schematically shown in figure 3, provides to introduce the cannula into the aorta, upstream of the aorta clamping or clamped region, that is at the aorta root, according to a standard procedure ( by performing a needle puncture, withdrawing the needle and suturing the flange).

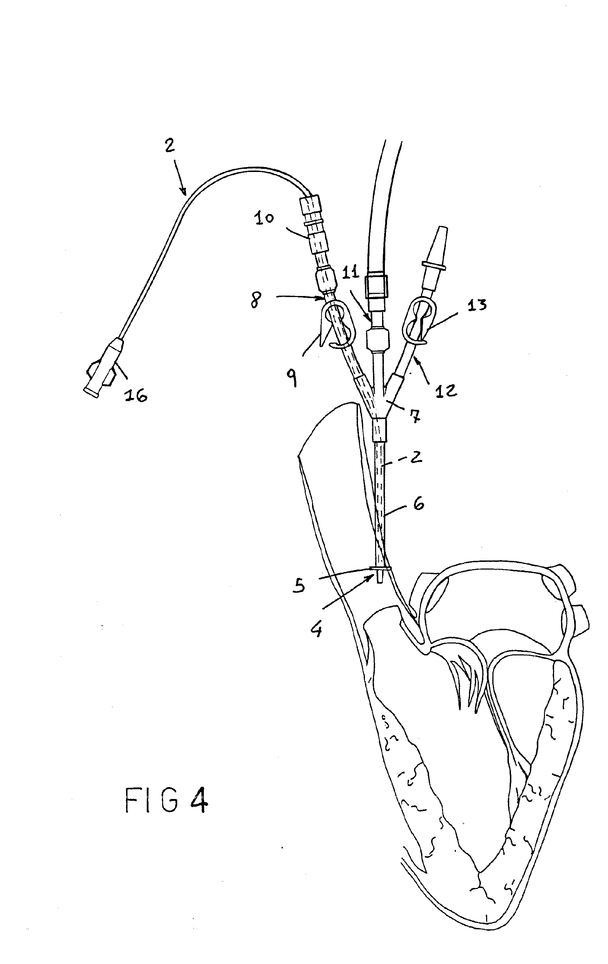

Upon having located the three-way cannula, as is shown in figure 4, the filling-in catheter may be introduced through the haemostatic valve and, upon connecting the cardioplegia solution line to the outer circuit, the vent line being always closed by its clamping element.

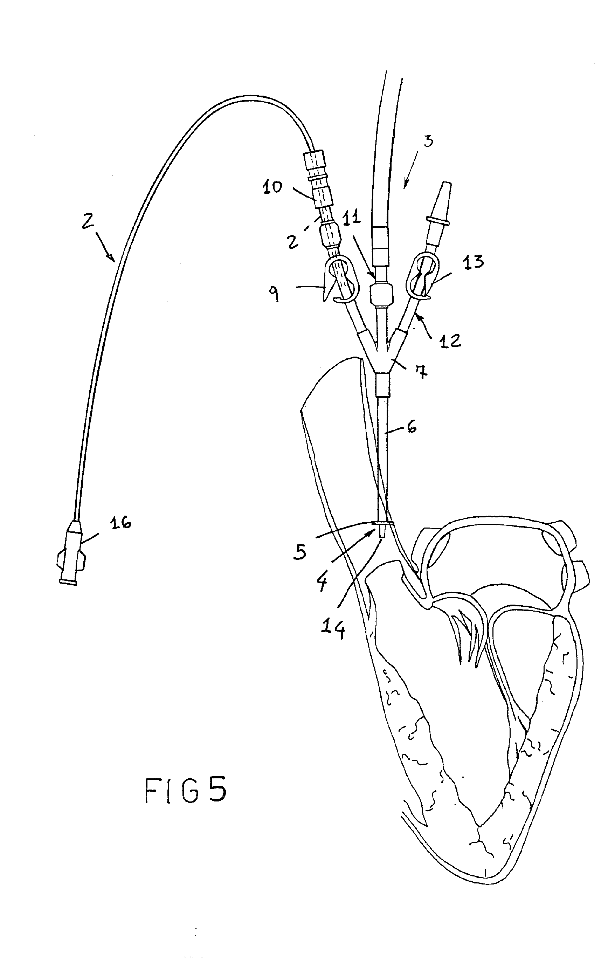

The catheter may pass through the cannula tip, or it can be withdrawn from the tip of the cannula, as is shown in figure 5, depending on the mode of operation preferred by the surgeon.

More specifically, as the diameter of the catheter is less than that of the cannula tip (for example 2 mm instead of 3 mm), the cardioplegia solution is perfectly perfused through the coronary arteries, notwithstanding the presence of the catheter occluding a portion of the lumen.

If the catheter and cannula tip have the same diameter (for example 2 mm), it is necessary to hold the catheter at a withdrawn position thereof.

In this condition, the catheter can be introduced only as it is required, and accordingly the cannula may comprise only two lumens, the catheter being introduced from the vent line.

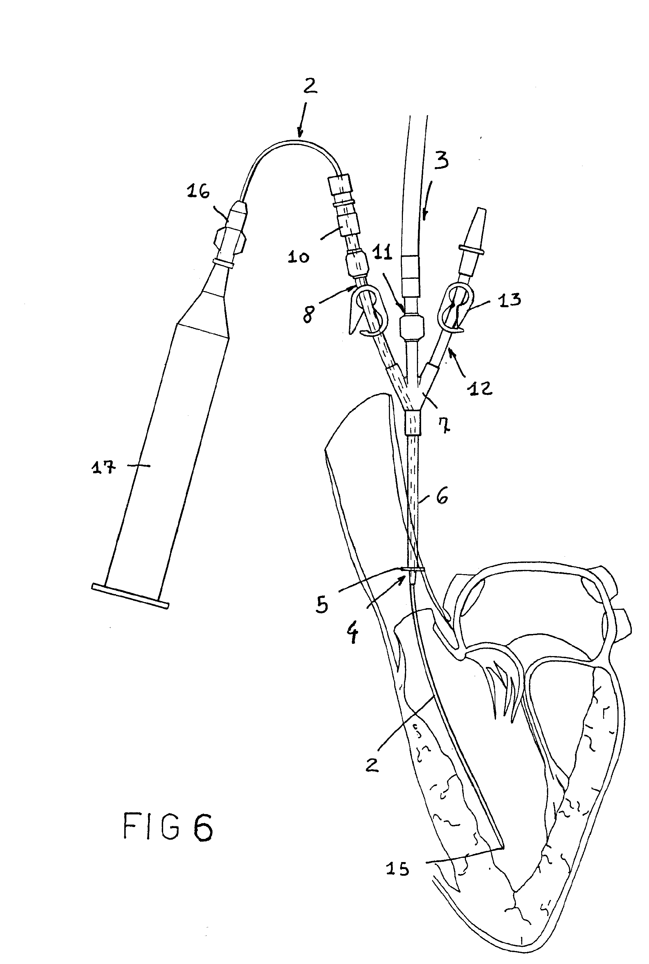

At the end of the mitral valve repairing plastics operation, since it is necessary to test the repaired mitral valve tightness, the catheter must be properly arranged in the ventricle, as shown in figure 6.

Thus, by using a standard syringe, it is possible to fill-in the ventricle with the physiologic solution.

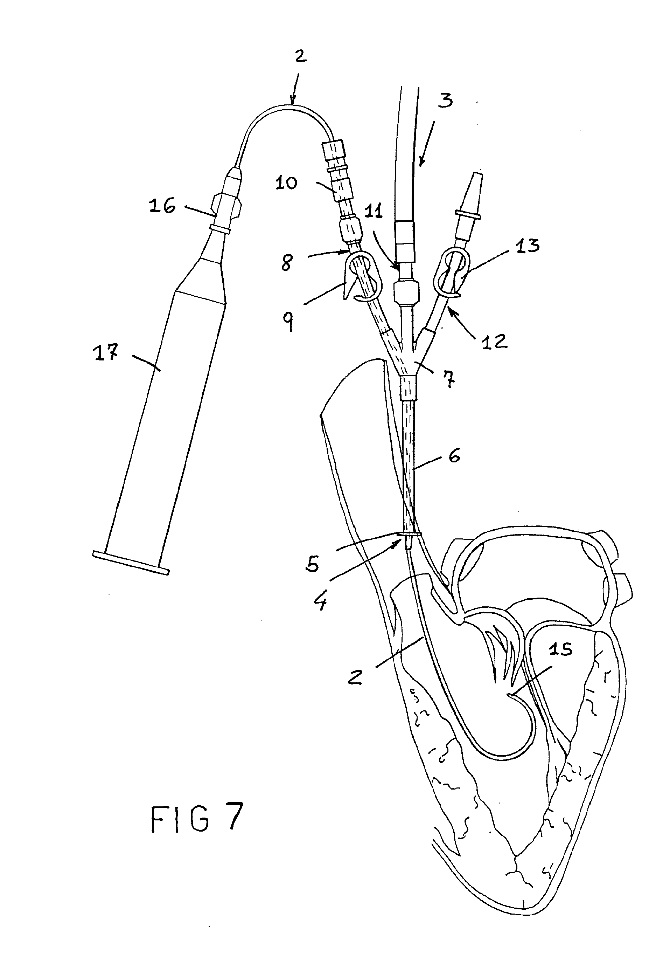

The catheter may be of a straight or pig-tail type, that is it may have either a straight or a curved tip portion, as indicated by the reference number 18 and shown in figure 7.

A pig-tail catheter being very useful for properly locating said catheter at the start of the CEC, since it cannot be accidentally withdrawn or disengaged.

The system further comprises a cardioplegia solution infusion cannula having one, two or three cannula lumens and with an aortic transvalve catheter for filling-in the left ventricle to test the mitral valve tightness at the end of the repairing plastics operation even if the single components would be separately supplied.

In this connection it should be pointed out that the three-way cannula may also be replaced by a cannula having a 2-way arrangement or a single-way arrangement provided that the vent feature is omitted.

Moreover, said cannula and catheter may be coated by heparine or other materials adapted to improve the system biocompatibility.

It has been found that the invention fully achieves the intended aim and objects.

In fact, the invention has provided a composite system allowing to anatomically test the tightness of a repaired mitral valve at the end of a mitral valve repairing plastics operation and during a CEC procedure, by filling-in the heart left ventricle.

The system according to the invention has the great advantage that the inventive device does not pass through the mitral valve and does not require a repeated aortotomy operation.

In particular, the device according to the present invention can be combined with the cannula for administering a cardioplegia solution through an anterograde way.

The use of the inventive device provides the following great advantages:
it is not necessary to provide several insertion places for inserting a catheter;
the repaired mitral valve test provides very reliable results, since the catheter does not pass through the valve;
the monitoring and testing operations, at the end of the repairing plastics procedure can be performed in a very quick manner;
the monitoring system can be used in very simple manner, and
further conventionally used devices are not changed.

In practicing the invention, the used materials, as well as the contingent size and shapes, can be any, depending on requirements.

## Claims

1. A composite device for controlling a mitral valve repairing plastics operation and performing a simultaneous cardioplegia solution infusion in a conservative surgical operation on a mitral valve, **characterized in that** said composite device comprises two coupled catheters, one for infusing a ventricle filling-in physiologic solution, and the other for providing an aorta anchoring and access tip means for different types of infusions, in particular a cardioplegia solution and ventricle filling-in infusion, said second catheter comprising an access cannula used as an aorta access element and including an implantable tip part, a flexible material cannula body, a fitting region for a three way access to said aorta, a ventricular filling-in line, a cardioplegia solution line connected to a cardioplegia solution circuit, a venting line, and an aorta puncturing needle occlusive with respect to said tip part.

2. A device according to claim 1, according to claim 1, **characterized in that** said tip part is made of a soft and flexible material, such as PVC, PU or the like, and comprises a suturing flange to suture said tip part to a vessel having a target diameter, for example 3 mm.

3. A device, according to claim 1, **characterized in that** said cannula body is made of a soft and flexible materials such as PVC, PU or the like, of a single-volume type, thereby allowing said cannula body to be directly connected to said tip part.

4. A device, according to claim 1, **characterized in that** the ventricle filling-in line comprises a clamping element and a haemostatic valve.

5. A device, according to claim 1, **characterized in that** said venting line comprises a venting line clamping element.

6. A device, according to claim 1, **characterized in that** the three ways of said cannula may be reversed and are adapted to operate depending on outer line connections, the cardioplegia solution line being arranged on a side of said cannula and the filling-in line having a straight configuration.

7. A device, according to claim 1, **characterized in that** said mitral valve testing catheter comprises an extruded single-volume soft and strong material element, made of polyethereblockamide, PU or the like, having a multilayer structure, a thin thickness and an atraumatic open tip having a same or less diameter than that of said cannula tip part, in particular 2 mm, and being perforated through a 35 mm length, said catheter also including insertion notches.

8. A device, according to claim 7, **characterized in that** said mitral valve test catheter is directly coupled to a physiologic solution infusion syringe by a luer connection.

9. A device, according to claim 7, **characterized in that** said mitral valve testing catheter has either a straight or a pig-tail configuration, said pig-tail configuration catheter including a curved tip.

10. A device, according to claim 1, **characterized in that** said cannula is adapted to be implanted the cannula into the aorta, upstream of an aorta clamped region, or in an aorta root region, by a standard procedure, such as performing a middle puncture, withdrawing the needle and suturing a flange, and, upon installing said three-way cannula, inserting said filling-in catheter through the haemostatic valve and coupling the cardioplegia solution line to an outer circuit, the vent line being always closed by a clamping element; said catheter being caused to pass through said cannula tip part or being withdrawn therefrom; as the diameter of said catheter is less than the diameter of the cannula tip part, said cardioplegia solution efficiently perfusing the coronary arteries; as said catheter and tip part have the same diameter, said catheter being held at a withdrawn position thereof to be introduced only as necessary from the vent line; at the end of the mitral valve repairing plastics operation, said catheter being introduced into said ventricle; by a standard syringe being performed a filling-in operation for filling-in the ventricle by the physiologic solution.

11. A device, according to claim 1, **characterized in that** said 3-way cannula may be replaced by a 2-way or a single-way configuration cannula.

12. A device, according to claim 1, **characterized in that** said cannula and catheter are coated by heparine or other materials adapted to improve the biocompatibility of said device.
